# EUROPÄISCHE PATENTSCHRIFT

(11) **EP 1 837 696 B1**
(45) Veröffentlichungstag und Bekanntmachung des Hinweises auf die Patenterteilung: **12.10.2016**
(21) Anmeldenummer: 06005655.3
(22) Anmeldetag: 20.03.2006
(51) Int. Cl.: G02B 26/10, G02B 3/14, A61F 9/008, B23K 26/04, B23K 26/06, G02B 27/00

(54) **Optisches Abbildungssystem und Verfahren zum Steuern sowie Verwendung eines solchen**
Optical imaging system and method for controlling and using such an imaging system
Système d'imagerie optique et procédé de commande, ainsi qu'utilisation d'un tel procédé

(43) Veröffentlichungstag der Anmeldung: 26.09.2007
(73) Patentinhaber: WaveLight GmbH, 91058 Erlangen (DE)
(72) Erfinder: Mrochen, Michael, 8193 Eglisau (CH); Donitzky, Christof, 90542 Eckental (DE); Vogler, Klaus, 99444 Blankenhain (DE)
(74) Vertreter: von Hellfeld, Axel

(56) Entgegenhaltungen:
- DE-A1- 3 711 905
- US-A- 5 418 638
- US-A- 5 980 513
- US-A- 6 072 176
- US-A1- 2001 031 960
- US-A1- 2004 243 111
- US-B1- 6 210 401
- PATENT ABSTRACTS OF JAPAN Bd. 2000, Nr. 09, 13. Oktober 2000 (2000-10-13) -& JP 2000 171742 A (CANON INC), 23. Juni 2000 (2000-06-23)
- PATENT ABSTRACTS OF JAPAN Bd. 015, Nr. 067 (P-1167), 18. Februar 1991 (1991-02-18) -& JP 02 289815 A (RICOH CO LTD), 29. November 1990 (1990-11-29)
- 'JP2000171742 A 20000623' 23 Juni 2000, Seite 10PP, XP055144517

## Beschreibung

Die Erfindung betrifft ein optisches Abbildungssystem mit mehreren Linsen, ein Verfahren zum Steuern eines optischen Abbildungssystems und die Verwendung eines solchen.

Lasersysteme mit kurzen Laserpulsen, zum Beispiel im Millisekunden- bis Femtosekundenbereich, haben vielfältige Anwendungen in der Materialbearbeitung und in der medizinischen Technik gefunden. Dabei sind insbesondere photoablative und photodisruptive Wirkungen der Laserstrahlung von Bedeutung.

Zur Steuerung der Laserstrahlung in einer Ebene, die in der Regel senkrecht steht zur optischen Achse des Abbildungssystems und die üblicherweise als x-y-Ebene bezeichnet wird, sind Abtasteinheiten (Scan-Systeme) bekannt, die zum Beispiel auf dem Galvanometerprinzip basieren.

Viele Anwendungen in der Materialbearbeitung und in der medizinischen Chirurgie erfordern eine hochpräzise Fokussierung mit geringen Abmessungen des Fokus, zum Beispiel eine Fokussierung in einem Bereich mit wenigen Mikrometern Durchmesser.

Die Fokussierung der Strahlung erfolgt typischerweise in der auf der x-y-Ebene senkrecht stehenden z-Achse, die in der Regel mit der optischen Achse des Abbildungssystems zusammenfällt. Eine Änderung der Lage des Fokus in z-Richtung wird im Stand der Technik in der Regel durch Verschiebung von Linsen entlang der z-Achse bewirkt, zum Beispiel durch Verschiebung eines Teleskops. In der Regel sind hier Genauigkeiten hinsichtlich der Lage des Fokus im Bereich von wenigen µm erforderlich.

Typische Anwendungen von Abtast-Lasersystemen, die eine Steuerung des Fokus (auch Spot genannt) in x-y-z-Richtungen beinhalten, sind in der Industrie die Photo-Lithographie, das Schneiden und Bohren von Metallen oder anderen Werkstoffen sowie das Gravieren durchsichtiger Materialien.

Typische Anwendungen solcher Lasersysteme mit dreidimensionaler Steuerung des Spot in der medizinischen Diagnostik sind die optische Kohärenztomographie, die konfokale Mikroskopie, die Ophthalmosopie und die adaptive Optik.

Typische Anwendungen von optischen Abbildungssystemen der Eingangs genannten Art mit Steuerung des Fokuspunktes in der x-y-Ebene und in der z-Achse in der medizinischen Therapie sind die refraktive Hornhautchirurgie mit zum Beispiel Excimerlasern, die intraokulare Chirurgie mit Femtosekunden-Lasern (z.B. beim LASIK-Schnitt), die Dermaabrasio, zum Beispiel mit Erbium:YAG-Lasern, oder auch die Netzhautphotokoagulation.

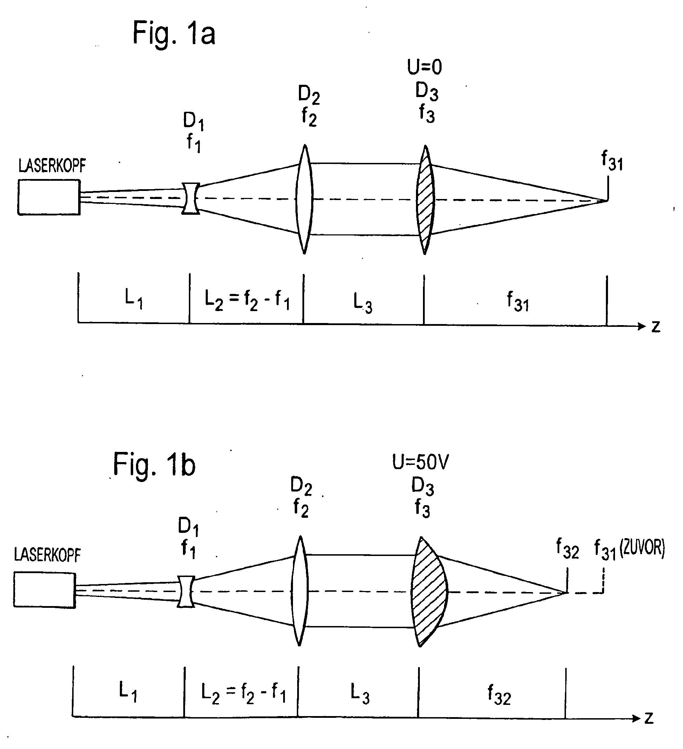

Die JP 2000-171742 offenbart ein optisches Abtastsystem, bei dem eine Lichtquelle, ein Kondensor und eine Abtasteinheit aufeinanderfolgend auf einer optischen Achse angeordnet sind, wobei die von der Lichtquelle ausgehende und an der Abtasteinheit abgelenkte Strahlung, eine Oberfläche abtastet (siehe Figur 1). Der Kondensor besteht aus einer Kondensorlinse und einer Linse, die in ihrem Fokus variabel ist. Bei der JP 2000-171742 befindet sich die Abtasteinheit bezogen auf die optische Achse der Vorrichtung an äußerster Position. Somit offenbart die JP 2000-171742 kein optisches Abbildungssystem, bei dem die Abtasteinheit im Strahlengang hinter einem Teleskop und vor einer Fokussierungslinse angeordnet ist, wobei eine Flüssigkeitslinse die Bildfeldwölbung einer Fokussierungslinse kompensiert.

Die JP 2-289815 beschreibt eine optische Abtastvorrichtung zur Entfernung von Feldkrümmungen in einer Abtastrichtung durch Bereitstellen einer Einrichtung zur Veränderung der Fokallänge. Dabei ist eine Linse, eine Linse, eine Abtasteinheit, eine Linse, und eine Fokussierungslinse entlang einer optischen Achse angeordnet. Die Abtasteinheit befindet sich nicht im Strahlengang hinter einem Teleskop und vor einer Fokussierungslinse. Die Kombination der beiden Linsen, hinter denen die Abtasteinheit angeordnet ist, kann nicht als ein Teleskop bezeichnet werden.

Das Dokument US 2004/0243111 A1 betrifft ein Lasersystem zur Bearbeitung von Gewebe. Das System umfasst eine Laserquelle zur Erzeugung eines Laserstrahls, der in eine Strahlaufweitungsoptik eingekoppelt wird. Anhand der Strahlaufweitungsoptik kann der Strahldurchmesser angepasst werden, wobei als Strahlaufweitungsoptik beispielsweise ein Teleskop mit einer Streulinse plus einer Sammellinse zum Einsatz kommt. Der aufgeweitete Laserstrahl wird mit Hilfe einer Strahlablenkungsvorrichtung, wie beispielsweise eines XY-Scanners, zu einer Strahlfokussierungsvorrichtung geführt, welche entlang der Z-Achse versetzbar ist und das Verschieben des Fokalpunktes erlaubt. Alternativ kann ein fokussieroptisches System mit variabler Brennweite zum Einsatz kommen, um die Fokusposition in Z-Richtung zu verschieben.

Die Erfindung hat das Ziel, für die vorgenannten Techniken, insbesondere die refraktive Hornhautchirurgie, Mittel bereitzustellen, mit denen in einfacher Weise und mit hoher Genauigkeit eine Fokussteuerung in x-, y- und z-Richtungen ermöglicht ist.

Dieses Ziel wird durch ein optisches Abbildungssystem gemäß Anspruch 1 erreicht.

Hierzu stellt die Erfindung ein optisches Abbildungssystem bereit mit mehreren Linsen, wobei mindestens eine der Linsen eine elektrisch einstellbare Flüssigkeitslinse ist, und mit einer Abtasteinheit in einer x-y-Ebene, wobei die Flüssigkeitslinse für eine Verschiebung des Brennpunktes der Abbildung in z-Richtung, die senkrecht steht auf der x-y-Ebene, eingerichtet ist.

Flüssigkeitslinsen sind als solche bekannt und beruhen auf dem Lippmann-Effekt (vgl. W. Mönch, W.F. Kromann, H. Zappe, "Variable Brennweite durch flüssige Mikrolinsen" in Photonik 4/2005, S. 44). Durch Anlegen einer Spannung verändert sich die Krümmung der Oberfläche einer Flüssigkeitslinse und damit ihre Brennweite. Insbesondere ermöglichen Flüssigkeitslinsen durch Variation der elektrischen Spannung eine Brechkraftänderung von einigen 10 dpt innerhalb von wenigen Millisekunden.

Gemäß einer Variante lehrt die Erfindung ein optisches Abbildungssystem mit mindestens einer Fokussierungslinse, mindestens zwei Linsen in einem Teleskop, wobei mindestens eine der Teleskoplinsen eine elektrisch einstellbare Flüssigkeitslinse ist, und mit einer Abtasteinheit in einer x-y-Ebene, die im Strahlengang hinter dem Teleskop und vor der Fokussierungslinse angeordnet ist, wobei die Flüssigkeitslinse die Bildfeldwölbung der Fokussierungslinse kompensiert.

Die Erfindung beinhaltet auch ein Verfahren zum Steuern eines optischen Abbildungssystems mit einer Abtasteinheit in einer x-y-Ebene und mit Fokussierungsmitteln zum Fokussieren der Abbildung in einer z-Richtung, die senkrecht steht auf der x-y-Ebene, dadurch gekennzeichnet, dass dem Steuerverfahren eine mathematische Funktion zugrunde liegt, welche die Lage des Brennpunktes in der z-Richtung als direkte Funktion der Position in der x-y-Ebene beinhaltet

Gemäß einer Variante beinhaltet die Erfindung ein Verfahren zum Steuern eines optischen Abbildungssystems gemäß einem Steuerprogramm, mit dem ein Brennpunkt der Abbildung in Richtung der Strahlung gesteuert wird, wobei die Lage des Brennpunktes gemessen und die Steuerung den Brennpunkt in Abhängigkeit von der Messung einstellt.

Insbesondere lehrt die Erfindung die Verwendung eines der vorstehend beschriebenen Abbildungssysteme und Verfahren in der Hornhautchirurgie, z.B. LASIK, unter Einsatz eines Femtosekunden-Lasers. Auch ist die Erfindung allgemein in der technologischen Materialbearbeitung einsetzbar.

Nachfolgend wird ein optisches Abbildungssystem anhand der Zeichnungen näher beschrieben.

Die Figuren 1a und 1b zeigen schematisch den Aufbau eines optischen Abbildungssystems, mit dem die Lage eines Fokus auf einer z-Achse einstellbar ist.

Beispielhaft soll das Abbildungssystem mit Blick auf den Einsatz eines Femtosekunden-Lasers (also eines gepulsten Lasers mit Pulslängen kürzer als eine Pikosekunde) in der LASIK-Hornhautchirurgie erläutert werden.

Ein Laserkopf emittiert die genannten kurzen Laserpulse und bewirkt zum Beispiel durch Photodisruption das Schneiden eines sogenannten Flaps in der Hornhaut. Hierzu wird die Laserstrahlung unter der Hornhautoberfläche im Stroma fokussiert und zeitlich-räumlich so gesteuert, dass die gewünschte Flapform geschnitten wird. Erforderlich ist hierzu eine 3-dimensionale Steuerung des Fokus der Laserpulse, also eine Steuerung in x-, y-, und z- Richtung. Die Steuerung in der x-y-Ebene, die in der Figur senkrecht auf der Zeichnungsebene steht und zu der die z-Achse, die auch die optische Achse des gezeigten Abbildungssystems darstellt, senkrecht steht, erfolgt mit herkömmlichen Mitteln, zum Beispiel mit dem Galvanometerprinzip, und ist hier nicht näher dargestellt. Insoweit kann auf den Stand der Technik zurückgegriffen werden.

Näher dargestellt ist die Veränderung des Fokus in der zur x-y-Ebene senkrecht stehenden z-Richtung, die in der Figur dargestellt ist.

Nachfolgend sind die in der Figur verwendeten Abkürzungen und die damit jeweils beschriebene physikalische Größe aufgeführt:

| Abkürzungen | Physikalische Größe |
|---|---|
| L1 | Abstand Laserkopf - Linse 1 |
| D1 | Durchmesser der Linse 1 |
| f1 | Brennweite der ersten Linse (Zerstreuung) |
| L2 | Abstand der beiden Linsen im Teleskop |
| D2 | Durchmesser der Linse 2 |
| f2 | Brennweite der 2. Linse |
| V | Vergrößerung |
| L3 | Abstand zwischen 2. und 3. Linse |
| D3 | Durchmesser der 3. Linse |
| f3 | Brennweite der 2. Linse |
| L4 | Abstand zwischen Linse 3 und Fokalpunkt |
| fs | Verschiebung des Fokalpunktes |

Mindestens eine der Linsen in der Abbildungsoptik ist eine elektrisch steuerbare Flüssigkeitslinse. Zum besseren Verständnis der Erfindung sei nachfolgend der - die Maßgabe des Anspruchs 1, wonach mindestens eine der Teleskoplinsen eine Flüssigkeitslinse sein soll, nicht erfüllende - Fall betrachtet, dass die Linse D3 eine solche Flüssigkeitslinse ist. Durch Änderung der Brennweite f3 dieser Linse kann die Lage des Fokuspunktes, d.h. der Abstand L4 zwischen der Linse D3 und dem Fokuspunkt, schnell und präzise variiert werden.

Figur la zeigt den Abbildungszustand ohne an die Flüssigkeitslinse D3 angelegte Spannung, während Figur 1b die Änderung der Position des Fokus in z-Richtung bei Anlegen einer Spannung U=50V an die Flüssigkeitslinse D3 zeigt.

Die Figuren 1a und 1b zeigen ein Aufweitteleskop, bei dem ohne an die Flüssigkeitslinse D3 angelegte Spannung (U=0V) gilt: f₃=f₃₁. Bei Anlegen einer Spannung U=50V gemäß Figur 1b gilt f₃=f₃₂<f₃₁.

Die Flüssigkeitslinse und die (in der Figur nicht näher gezeigte) Abtasteinheit in der x-y-Ebene werden von einem Rechner gemäß einem Steuerprogramm gesteuert. Bei Erzeugung eines Flaps im LASIK-Verfahren wird das Steuerprogramm so angelegt, dass jeder Position in der x-y-Ebene (wobei die Ebene in Punkte diskretisiert ist) eine Position in der z-Achse zugeordnet ist. Durch diesen direkten funktionalen Zusammenhang zwischen der x-y-Position und der z-Position ist das Steuerprogramm einfach darstellbar.

Eine nicht näher in der Figur dargestellte Weiterung sieht vor, dass die z-Position des Fokus mit einer Messeinrichtung überwacht wird. Die Steuerung erfolgt dann online derart, dass die Messung direkt in das Steuerprogramm eingeht und bei unerwünschter Verschiebung des Fokus in der z-Achse die Flüssigkeitslinse entsprechend zur Korrektur angesteuert wird.

## Patentansprüche

1. Optisches Abbildungssystem mit:
- mindestens einer Fokussierungslinse,
- mindestens zwei Linsen in einem Teleskop,
- einer Abtasteinheit in einer x-y-Ebene,
**dadurch gekennzeichnet, dass** mindestens eine der Teleskoplinsen eine elektrisch einstellbare Flüssigkeitslinse ist und die Abtasteinheit im Strahlengang hinter dem Teleskop und vor der Fokussierungslinse angeordnet ist, wobei das Abbildungssystem dazu eingerichtet ist, durch elektrische Einstellung der Flüssigkeitslinse die Bildfeldwölbung der Fokussierungslinse zu kompensieren.

2. Verfahren zum Steuern eines optischen Abbildungssystems nach Anspruch 1, wobei die mindestens eine Fokussierungslinse zum Fokussieren der Abbildung in einer z-Richtung eingerichtet ist, die senkrecht steht auf der x-y-Ebene, wobei dem Steuerverfahren eine mathematische Funktion zugrunde liegt, welche die Lage des Brennpunktes in der z-Richtung als direkte Funktion der Position in der x-y-Ebene beinhaltet.

3. Verfahren zum Steuern eines optischen Abbildungssystems nach Anspruch 1 gemäß einem Steuerprogramm, mit dem ein Brennpunkt der Abbildung in Richtung der Strahlung gesteuert wird, wobei die Lage des Brennpunktes gemessen und die Steuerung den Brennpunkt in Abhängigkeit von der Messung einstellt.

4. Verwendung eines optischen Abbildungssystems gemäß Anspruch 1 mit einem Femtosekunden-Laser in der Hornhautchirurgie.

5. Verwendung eines optischen Abbildungssystems gemäß Anspruch 1 in der Materialbearbeitung.

6. Verfahren gemäß einem der Ansprüche 2 oder 3 mit Verwendung eines Femtosekunden-Lasers in der Hornhautchirurgie.

7. Verfahren gemäß einem der Ansprüche 2 oder 3 zur Verwendung in der Materialbearbeitung.

## Claims

1. An optical imaging system, comprising:
- at least one focusing lens,
- at least two lenses in a telescope,
- a scanning unit in an x-y plane,
**characterized in that** at least one of the telescopic lenses is an electrically adjustable liquid lens and the scanning unit is arranged in the optical path behind the telescope and in front of the focusing lens, wherein the imaging system is adapted to compensate for the image field curvature of the focusing lens by electrical adjustment of the liquid lens.

2. A method for controlling an optical imaging system according to claim 1, wherein the at least one focusing lens is arranged for focusing the image in a z-direction, which is perpendicular to the x-y plane, wherein the control method is based on a mathematical function which comprises the position of the focal point in the z-direction as a direct function of the position in the x-y plane.

3. A method for controlling an optical imaging system as defined in claim 1 according to control program by means of which a focal point of the image is controlled in the direction of the radiation, wherein the position of the focal point is measured and the controller adjusts the focal point on the basis of the measurement.

4. Use of an optical imaging system according to claim 1 with a femtosecond laser in corneal surgery.

5. Use of an optical imaging system according to claim 1 in material processing.

6. The method according to any of claims 2 or 3, using a femtosecond laser in corneal surgery.

7. The method according to any of claims 2 or 3 for use in material processing.

## Revendications

1. Système d'imagerie optique comprenant :
- au moins une lentille de focalisation,
- au moins deux lentilles dans un télescope,
- une unité de balayage dans un plan x-y,
**caractérisé en ce qu'**au moins une des lentilles du télescope est une lentille liquide réglable électriquement et l'unité de balayage est disposée derrière le télescope et devant la lentille de focalisation dans le trajet des rayons, le système d'imagerie étant agencé pour compenser la courbure du champ d'image de la lentille de focalisation par réglage électrique de la lentille liquide.

2. Procédé de commande d'un système d'imagerie optique selon la revendication 1, dans lequel ladite au moins une lentille de focalisation est agencée pour focaliser l'image dans une direction z qui est perpendiculaire au plan x-y, le procédé de commande étant basé sur une fonction mathématique qui contient la position du foyer dans la direction z comme fonction directe de la position dans le plan x-y.

3. Procédé de commande d'un système d'imagerie optique selon la revendication 1 d'après un programme de commande, avec lequel un foyer de l'image est commandé dans la direction du rayonnement, la position du foyer étant mesurée et la commande réglant le foyer en fonction de la mesure.

4. Utilisation d'un système d'imagerie optique selon la revendication 1 avec un laser femtoseconde dans la chirurgie de la cornée.

5. Utilisation d'un système d'imagerie optique selon la revendication 1 dans l'usinage de matière.

6. Procédé selon l'une des revendications 2 ou 3 avec utilisation d'un laser femtoseconde dans la chirurgie de la cornée.

7. Procédé selon l'une des revendications 2 ou 3 destiné à être mis en oeuvre dans l'usinage de matière.
